Europäisches Patentamt

(19) European Patent Office

Office européen des brevets

(11) Numéro de publication: **0 350 390**
**A1**

## DEMANDE DE BREVET EUROPEEN

(21) Numéro de dépôt: **89401914.0**

(22) Date de dépôt: **04.07.89**

(51) Int. Cl.⁵: **A 61 B 17/58**

(30) Priorité: **07.07.88 FR 8809202**

(43) Date de publication de la demande:
**10.01.90 Bulletin 90/02**

(84) Etats contractants désignés:
**CH DE ES GB IT LI SE**

(71) Demandeur: **MICHAEL'S FRANCE Société à Responsabilité Limitée dite**
**55, Avenue Sainte-Foy**
**F-92200 Neuilly (FR)**

(72) Inventeur: **Aghion, Michael**
**44, rue de l'Alma**
**F-92400 Courbevoie (FR)**

(74) Mandataire: **Dawidowicz, Armand Cabinet Lemonnier-Dawidowicz**
**4, Boulevard Saint Denis**
**F-75010 Paris (FR)**

(54) Clou intramédullaire universel, en particulier pour le fémur.

(57) L'invention concerne un clou intramédullaire universel, en particulier pour le fémur.

Le clou intramédullaire selon l'invention est caractérisé par le fait qu'il est constitué par au moins un profilé (1) à section (2) constante déformable élastiquement transversalement, ladite section (2) étant, au repos, sensiblement inscrite dans une circonférence (3) et ayant la forme générale d'un U occupant plus de la moitié de ladite circonférence (3), une extrémité de ladite section (2) étant terminée par un élargissement circulaire (4) et l'autre extrémité de ladite section (2) étant terminée par un élargissement en forme de pince (5) dont la cavité (6) est limitée par un arc de cercle complémentaire dudit élargissement circulaire (4). Application à la chirurgie.

FIG.2

Bundesdruckerei Berlin

EP 0 350 390 A1

**Description**

## CLOU INTRAMEDULLAIRE UNIVERSEL, EN PARTICULIER POUR LE FEMUR.

L'invention concerne un clou intramédullaire universel, en particulier pour le fémur.

Les clous intramédullaires sont utilisés en chirurgie osseuse pour l'obturation d'un canal ou d'une cavité médullaire. Compte tenu des diamètres et longueurs très différents de la zone de canal ou de la cavité à obturer, selon le type d'os, l'âge du patient et la région de l'os concernés, il est nécessaire de disposer d'un clou médullaire de diamètre et de longueur déterminés pour l'opération, ce qui conduit à maintenir une gamme très importante de clous, de l'ordre de 250 modèles environ. La gestion et l'utilisation d'un tel stock sont évidemment compliqués et onéreux.

En outre, les clous connus ont souvent une résistance mécanique insuffisante, en particulier en compression longitudinale, ce qui est préjudiciable aussi bien lors de la mise en place qu'après pose. Ces clous connus ne permettent pas un ancrage dans les parois du canal ou de la cavité médullaire, ce qui limite leurs applications.

La présente invention vise à pallier les inconvénients des clous médullaires connus grâce à un nouveau clou d'usage universel, ne nécessitant donc la présence en stock que d'un seul modèle, qui soit de résistance mécanique suffisante, en particulier en compression longitudinale, tout en présentant la flexibilité transversale requise, et qui permette un ancrage dans le canal ou la cavité médullaire.

A cet effet, le clou intramédullaire selon l'invention est caractérisé par le fait qu'il est constitué par au moins un profilé à section constante déformable élastiquement transversalement, ladite section étant, au repos, sensiblement inscrite dans une circonférence et ayant la forme générale d'un U occupant plus de la moitié de ladite circonférence, une extrémité de ladite section étant terminée par un élargissement circulaire et l'autre extrémité de ladite section étant terminée par un élargissement en forme de pince dont la cavité est limitée par un arc de cercle complémentaire dudit élargissement circulaire.

La forme et l'élasticité du clou selon l'invention permettent des modes d'utilisation très variés, selon le diamètre de la section à obturer. Pour les petits diamètres, le profilé peut être utilisé seul, son élasticité transversale permettant un rapprochement des bords, constitués par les élargissements de sa section transversale, pour obtenir le diamètre voulu. Le clou est en général composé de plusieurs profilés identiques qui peuvent être assemblés soit concentriquement, par section identique, soit tête-bêche, par section inverse, la surépaisseur cylindrique d'un profilé étant logée dans la cavité de la pince d'un autre profilé.

Avantageusement, afin de permettre un ancrage quand cela est nécessaire, au moins un des bords de la cavité de l'extrémité en forme de pince est à arête vive.

Le profilé selon l'invention est découpé à la longueur voulue au moyen d'une pince constituée par un outil - support du profilé, une lame de coupe articulée sur ledit outil-support et munie d'un bloc de coupe, et un levier d'attaque articulé sur ledit outil - support et comportant un axe d'appui sur ladite lame de coupe.

Pour faciliter le choix de la longueur, le profilé comporte un marquage de longueur.

Avantageusement, le profilé est muni de trous équidistants, qui servent à l' extraction du clou , en étant certain de toujours disposer d'un tel trou au voisinage de l'extrémité libre, après découpe à longueur.

Le profilé peut être métallique, de préférence en acier inoxydable. On peut également utiliser des alliages cobalt-chrome-molybdème, du titane, des alliages d' aluminium, etc. Le profilé métallique est avantageusement produit par tréfilage.

Il peut également être fabriqué par extrusion d'une matière synthétique armée de fibres longitudinales, par exemple de fibres de verre, de carbone, de kevlar, etc.

Pour faciliter l'introduction du clou, on peut prévoir que les deux extrémités du profilé comportent un chanfrein formant un cône allongé. Une extrémité conique d'entrée reste disponible après découpe à longueur et la portion restante peut, selon sa longueur, être utilisée pour une autre opération, avec une extrémité conique d'entrée.

L'invention sera bien comprise à la lecture de la description suivante faite en se référant au dessin annexé dans lequel:

- la figure 1 est une vue schématique en plan d'un profilé selon un exemple de réalisation de l'invention;

- la figure 2 est une vue en coupe transversale du profilé de la figure 1;

- la figure 3 est une vue en élévation latérale d'une pince de coupe du profilé des figures 1 et 2, et

- la figure 4 est une vue en coupe transversale de la pince de la figure 3.

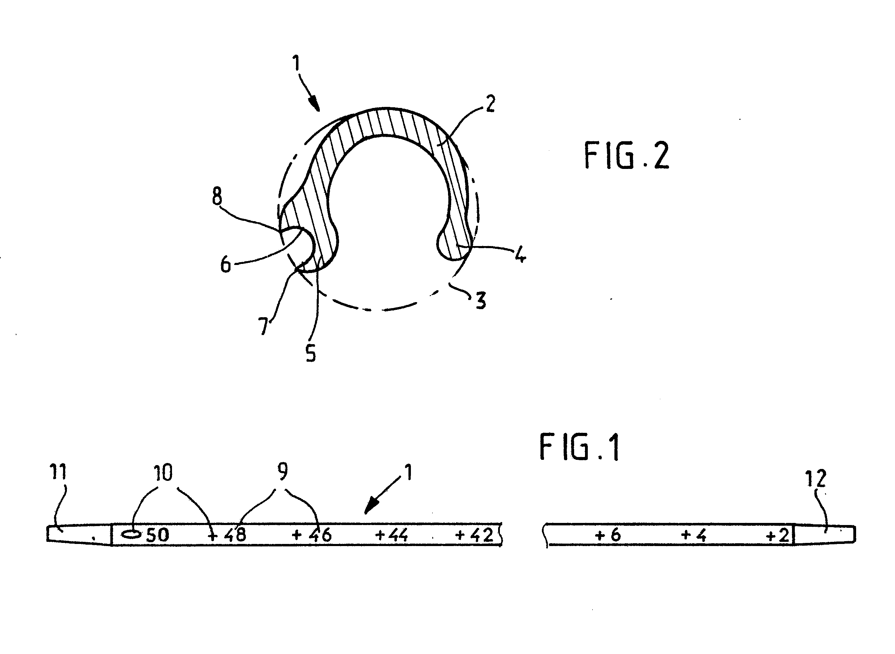

Conformément à la présente invention, un clou intramédullaire est formé d'au moins un profilé 1 dont un exemple de réalisation est illustré aux figures 1 et 2. Le profilé 1 a une section 2 constante, déformable élastiquement transversalement, qui est, dans la position de repos représentée à la figure 2, inscrite à l'intérieur d'une circonférence 3. La section 2 a la forme générale d'un U occupant plus de la moitié de la circonférence 3. Une extrémité de la section 2 est terminée par un élargissement circulaire 4 et l'autre extrémité est terminée par un élargissement en forme de pince 5. La cavité 6 de la pince 5 est formée par un arc de cercle de même rayon que la circonférence de l'élargissement circulaire 4, c'est-à-dire que cette cavité 6 est complémentaire de l'élargissement circulaire 4.

Dans l'exemple décrit, les pointes 7 et 8 de la pince 5 sont à arête vive, ce qui permet un ancrage du clou dans la paroi osseuse de la cavité médullaire où il est implanté.

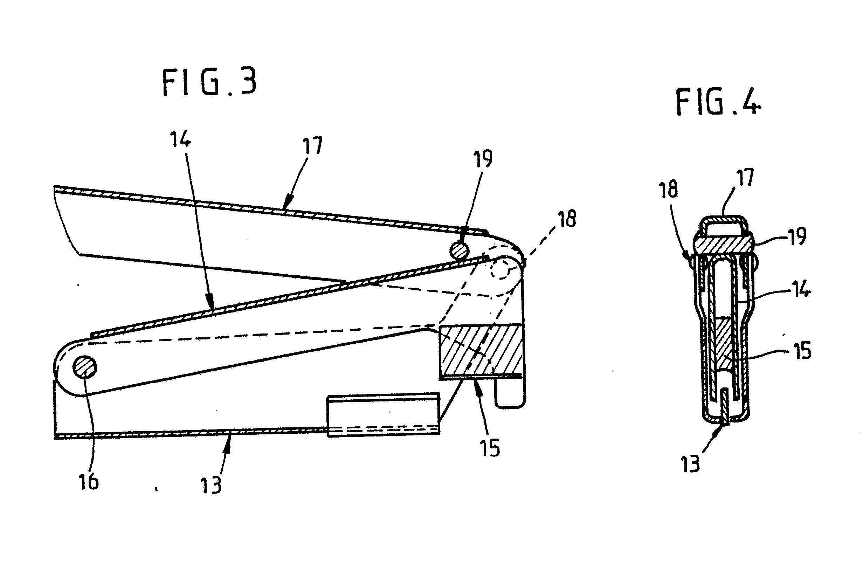

Le profilé 1 est muni d'un marquage de longueur 9 et comporte des trous oblongs 10 équidistants. Après découpe à la longueur choisie au moyen d'une pince qui sera décrite en relation avec les figures 3 et 4, un trou 10 est toujours voisin de l'extrémité coupée et peut être utilisé pour l'extraction du clou au moyen d'un crochet (non représenté).

En outre, les deux extrémités 11,12 du profilé 1 sont, dans l'exemple représenté, usinées avec un chanfrein formant un cône allongé facilitant l'introduction du clou. Après découpe à longueur du profilé 1, la portion subsistante peut, quand elle a une longueur suffisante, être utilisée avec le cône d'introduction 11,12 restant.

Le profilé peut être en métal, de préférence en acier inoxydable. Il peut également être en un alliage cobalt-chromemolybdème, en titane, en alliage d'aluminium ou tout autre métal ou alliage métallique convenable. Le profilé métallique peut être obtenu par tréfilage.

Le profilé 1 peut également être en matière synthétique armée de fibres longitudinales à module d'élasticité élevé telles que fibres de verre, de carbone, de kevlar, etc. Il est avantageusement obtenu par extrusion dans une filière de profil convenable. Il peut être radiopaque afin de permettre un examen radiographique non perturbé.

Pour l'obturation de petites cavités dont la section est inférieure à la circonférence 3, le clou est constitué d'un seul profilé 1 comprimé radialement en rapprochant ses élargissements d'extrémité 4 et 5.

Pour les cavités d'une section supérieure à la circonférence 3, on utilisera deux profilés 1 ou plus. Les profilés 1 peuvent alors être placés concentriquement en étant encastrés successivement les uns dans les autres. Selon un autre forme d'utilisation, ils sont montés tête-bêche, c'est-à-dire que l'élargissement à section circulaire 4 d'un profilé 1 est logé dans la cavité 6 de l'élargissement 5 du profilé 1, et ainsi de proche en proche. On obtient ainsi un assemblage par règlette cylindrique qui autorise une liberté angulaire favorable à la création des diamètres circonscrits au clou composite. Dans tous les cas, l'élasticité radiale des profilés 1 permet d'épouser les différentes sections progressives de la cavité intramédullaire.

Après mise à longueur, l'extrémité coupée présente une section 2 complète, ce qui facilite l'impact de la masse sur le clou à enfoncer.

La multiplicité des profilés 1 constituant le clou est favorable à une bonne tenue mécanique du fait de la liberté d'assemblage de chaque profilé n'ayant aucune contrainte longitudinale.

Lorsque le profilé 1 est métallique, le marquage de longueur 9 peut être effectué au laser. DAns le cas d'un profilé 1 plastique, on peut utiliser un encrage.

La forme de la section 2 n'est pas limitée à l'exemple représenté. Elle peut en particulier comporter des facettes longi tudinales planes.

Avec le clou selon l'invention, en plus de sa parfaite adaptation à la cavité intramédullaire, l'utilisation du profilé 1 de base est facilitée par le fait que l'utilisateur n'a qu'à choisir le nombre de profilés 1, identiques, en fonction de la dimension transversale de la cavité à obturer, et à les couper à la longueur voulu.

Cette mise à longueur est facilitée par l'utilisation de l'outil spécial représenté aux figures 3 et 4, qui fait partie de l'invention. Cet outil est constitué d'un support 13 du profilé 1, d'une lame de coupe 14 munie d'un bloc de coupe 15 pivotant par rapport au support 13 autour d'un axe 16, et d'un levier d'attaque 17 articulé sur le support 13 autour d'un axe 18 et qui comporte un axe d'appui 19 sur la lame 14. L'outil peut être actionné manuellement, hydrauliquement, pneumatiquement, électriquement, etc.

## Revendications

1.-Clou intramédullaire universel, en particulier pour le fémur, caractérisé par le fait qu'il est constitué par au moins un profilé (1) à section (2) constante déformable élastiquement transversalement, ladite section (2) étant, au repos, sensiblement inscrite dans une circonférence (3) et ayant la forme générale d'un U occupant plus de la moitié de ladite circonférence (3), une extrémité de ladite section (2) étant terminée par un élargissement circulaire (4) et l'autre extrémité de ladite section (2) étant terminée par un élargissement en forme de pince (5) dont la cavité (6) est limitée par un arc de cercle complémentaire dudit élargissement circulaire (4).

2.-Clou intramédullaire selon la revendication 1, caractérisé par le fait qu'au moins un des bords (7,8) de la cavité (6) de l'extrémité en forme de pince (5) est à arête vive.

3.-Clou intramédullaire selon l'une des revendications 1 et 2, caractérisé par le fait que le profilé (1) comporte un marquage de longueur (9).

4.-Clou intramédullaire selon l'une des revendications 1 à 3, caractérisé par le fait que le profilé (1) est muni de trous équidistants (10).

5.- Clou intramédullaire selon l'une des revendications 1 à 4, caractérisé par le fait que ledit profilé (1) est métallique.

6.-Clou intramédullaire selon la revendication 5, caractérisé par le fait que ledit profilé métallique (1) est obtenu par tréfilage.

7.- Clou intramédullaire selon l'une des revendications 1 à 4, caractérisé par le fait que ledit profilé (1) est en matière synthétique armée de fibres longitudinales à module d'élasticité élevé.

8.-Clou intramédullaire selon la revendication 7, caractérisé par le fait que ledit profilé (1) en matière synthétique armée est obtenu par extrusion.

9.- Clou intramédullaire selon l'une des revendications 1 à 8, caractérisé par le fait que les deux extrémités (11,12) du profilé (1) comportent un chanfrein formant un cône allongé.

10.- Clou intramédullaire selon l'une des revendications 7 et 8, caractérisé par le fait que le profilé (1) est en un matériau radiopaque.

11.- Clou intramédullaire selon l'une des revendications 1 à 10, caractérisé par le fait qu'il comprend au moins deux profilés (1) disposés concentriquement.

12.- Clou intramédullaire selon l'une des revendications 1 à 10, caractérisé par le fait qu'il comprend au moins deux profilés (1), l'élargissement à section circulaire (4) d'un profilé (1) étant logé dans la cavité (6) de l'élargissement en forme de pince (5) d'un profilé (1) adjacent.

13.- Outil de coupe d'un clou selon l'une des revendications 1 à 12, caractérisé par le fait qu'il est constitué par un outil-support (13) du profilé (1), une lame de coupe (14) articulée sur ledit outil-support (13) et munie d'un bloc de coupe (15), et un levier d'attaque (17) articulé sur ledit outil-support (13) et comportant un axe d'appui (19) sur ladite lame de coupe (14).

FIG.2

FIG.1

EP 0 350 390 A1

FIG.3

FIG.4

EP 0 350 390 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A- 893 401 (POHL) <br> * Page 1, lignes 42-48; page 2, lignes 23-33,72-86; page 4, lignes 3-33; page 6, lignes 13-30; figures 1,9,11,22,25,26,34-38,46,47 * <br> --- | 1,5,6,9 ,11,12 | A 61 B 17/58 |
| A | DE-C- 767 879 (POHL) <br> * Page 2, lignes 43-65; figures * <br> --- | 1,11,12 | |
| A | US-A-2 265 208 (THOMPSON) <br> * Page 2, colonne 1, lignes 50-57; page 2, colonne 1, ligne 74 - colonne 2, ligne 39; figures 1,2 * <br> --- | 3-5,10 | |
| A | US-A-3 893 196 (HOCHMAN) <br> * Colonne 2, lignes 47-65; figures 2,3 * <br> --- | 7 | |
| A | DE-C- 824 239 (SACHSE) <br> * Page 2, lignes 51,52; figure 4 * <br> --- | 9 | |
| A | US-A-4 682 590 (KOTHMANN) <br> * Colonne 3, lignes 48,49; figure 32 * <br> --- | 13 | DOMAINES TECHNIQUES RECHERCHES (Int. Cl.5) <br><br> A 61 B |
| A | DE-C- 913 228 (POHL) <br> --- | | |
| A | FR-A-1 031 128 (CARRIERI) <br> ----- | | |

Le présent rapport a été établi pour toutes les revendications

| Lieu de la recherche | Date d'achèvement de la recherche | Examinateur |
|---|---|---|
| LA HAYE | 13-10-1989 | KLEIN C. |

EPO FORM 1503 03.82 (P0402)